# EUROPEAN PATENT SPECIFICATION

(11) **EP 4 259 110 B1**
(45) Date of publication and mention of the grant of the patent: **09.07.2025**
(21) Application number: 21815540.6
(22) Date of filing: 30.11.2021
(51) Int. Cl.: A61K 31/00, A61K 47/00, A61K 8/11, A61K 8/34, A61K 8/73, A61Q 19/00, A23L 27/10, A23L 33/105, A61K 9/00, A61K 9/06, A61K 9/19, A61K 9/51, A61K 31/05, A61P 17/00

(54) **COMPOSITION COMPRISING SEA WATER AND CANNABINOID LOADED SUBMICROPARTICLES FOR PHARMACEUTICAL, NUTRACEUTICAL AND COSMETIC APPLICATIONS**
ZUSAMMENSETZUNG MIT MEERWASSER UND CANNABINOIDBELADENEN SUBMIKROPARTIKELN FÜR PHARMAZEUTISCHE, NUTRAZEUTISCHE UND KOSMETISCHE ANWENDUNGEN
COMPOSITION COMPRENANT DE L'EAU DE MER ET DES SUBMICROPARTICLES CHARGÉES EN CANNABINOÏDES POUR DES APPLICATIONS PHARMACEUTIQUES, NUTRACEUTIQUES ET COSMÉTIQUES

(30) Priority: 09.12.2020 EP 20383071
(43) Date of publication of application: 18.10.2023
(73) Proprietor: I+Med S. Coop., 01510 Vitoria-Gasteiz (Álava) (ES)
(72) Inventor: GARTZIANDIA LÓPEZ DE GOIKOETXEA, Oihane, 01510 VITORIA- GASTEIZ (Álava) (ES); ALONSO CARNICERO, José, María, 01510 VITORIA- GASTEIZ (Álava) (ES); PÉREZ GONZÁLEZ, Raúl, 01510 VITORIA- GASTEIZ (Álava) (ES); MUÑOZ MORENTIN, Manuel, 01510 VITORIA- GASTEIZ (Álava) (ES)
(74) Representative: Herrero & Asociados, S.L.
(86) International application number: PCT/EP2021/083550
(87) International publication number: WO 2022/122471

(56) References cited:
- WO-A1-2016/154032
- US-A1- 2015 064 250
- POTAS JOANNA ET AL: "Challenges in developing of chitosan - Based polyelectrolyte complexes as a platform for mucosal and skin drug delivery", EUROPEAN POLYMER JOURNAL, vol. 140, 1 November 2020 (2020-11-01), GB, pages 110020, XP055807799, ISSN: 0014-3057, DOI: 10.1016/j.eurpolymj.2020.110020
- TURCSÁNYI ÁRPÁD ET AL: "Chitosan-modified hyaluronic acid-based nanosized drug carriers", INTERNATIONAL JOURNAL OF BIOLOGICAL MACROMOLECULES, ELSEVIER BV, NL, vol. 148, 15 January 2020 (2020-01-15), pages 218 - 225, XP086077276, ISSN: 0141-8130, [retrieved on 20200115], DOI: 10.1016/J.IJBIOMAC.2020.01.118

## Description

### Field of the invention

The present invention relates generally to formulations of cannabinoids. In particular, the invention relates to a composition comprising cannabinoid loaded polymer submicroparticles soluble in aqueous media, more specifically a composition comprising sea water and phytocannabinoid loaded chitosan/hyaluronic acid submicroparticles, the method for the manufacture of this composition and its uses in pharmaceutical, nutraceutical and cosmetic applications.

### Background of the invention

Skin diseases represent the fourth leading source of non-fatal disease burden at global level *(P*. Wolkenstein et al. French People and Skin Diseases: Results of a Survey Using a Representative Sample. Archives of Dermatology,139 (2003) 1614-1619*)* and they are considered a major public health problem *(*R. J. Hay et al. The Global Burden of Skin Disease in 2010: an Analysis of the Prevalence and Impact of Skin Conditions. J. Investig. Dermatol. 134 (2014) 1527-1534). Moreover, skin conditions affect to a significant percentage of the adult population. Up to 20% of the adult population in west-European countries suffer from acne whereas atopic dermatitis and psoriasis affect up to 8% and 5% of the adult population respectively *(*A. Svenson et al. Prevalence of Skin Disease in a Population-Based Sample of Adults From Five European Countries. Br. J. Dermatol. 178(2018)1111-1118).

Acne, atopic dermatitis and psoriasis are related to alterations in the physiology and biochemical paths of the skin. Trace amounts of chemical elements are essential in skin metabolism for membrane function, immune modulation and enzyme co-factors. Natural sea water contains a variety of inorganic salts in a combined concentration close to 35 g/kg. Application of sea water to the skin is commonly used for the healing of those skin conditions and the acceleration of the skin repair process *(*H. Matz et al. Balneotherapy in Dermatology. Dermatol. Ther. 16 (2003) 132-140)*.* Less common skin diseases as pruritus, lichen rubra planus, rosacea and ichthyoses are also treated with highly concentrated salt solutions (L. Andreassi et al. Mineral Water and Spas in Italy. Clin. Dermatol 14 (1996) 627-632*;* J. Shani et al. Indications, Contraindications and Possible Side Effects of Climato Therapy at the Dead Sea. Int. J. Dermatol. 36 (1997) 481-492)

Nasal washes with seawater are indicated both for routine hygiene and certain pathologies, such as allergic rhinitis, dry rhinitis, rhinosinusitis or sinonasal polyposis, among others (K. E. Hermelingmeier et al. Nasal Irrigation as an Adjunctive Treatment in Allergic Rhinitis: a Systematic Review and Meta-analysis. Am. J. Rhinol. Allergy 26 (2012) e119-e125*;* P.-L. Bastier et al. Nasal irrigation: From Empiricism to Evidence-based Medicine. A review. European Annals of Otorhinolaryngology, Head and Neck Diseases. 132 (2015) 281-285*).* These washes hydrate the mucosa, fluidize secretions and eliminate impurities, pathogens and allergens, improving nasal congestion conditions. In addition, sea water facilitates the absorption of marine trace elements through the nasal mucosa and the vibration of the cilia of surface cells. The vibration movement behaves like a brush that sweeps and pushes mucus outwards.

**Hyaluronic acid** is a major component of the extracellular matrix (ECM) and thus is the major physiological constituent of the articular cartilage matrix and is particularly abundant in synovial fluid and in the skin.

The hyaluronic acid, in its acid or salt form, is a biomaterial broadly employed as an injectable material for applications in tissue engineering and especially for augmentation of skin tissue and of other soft tissues.

Hyaluronic acid is a linear non-sulfated glycosaminoglycan biopolymer composed of repeating units of D-glucuronic acid and N-acetyl-D-glucosamine (R. Tammi, U. M. Agren, A. L. Tuhkanen, M. Tammi. Hyaluronan metabolism in skin. Progress in Histochemistry & Cytochemistry 29 (1994) 1.-81*)*. At physiological pH (7.4) it is in the conjugate base hyaluronate form.

Hyaluronic acid is mostly synthesized in the skin by dermal fibroblasts and epidermal keratinocytes (*R. Tammi R., et al. 1994)* and acts as a water pump for maintaining the elasticity of the skin.

The ECM is composed of structural proteins such as collagen and elastin and of water, minerals and proteoglycans. This matrix is a dynamic structure with a structural role that provides to the skin with its mechanical properties of elasticity, firmness and tone.

Concerning the skin, it is noticed that, with age, the amount of hyaluronic acid and its degree of polymerization diminishes, causing a decrease in the amount of water retained in the connective tissue. In the meantime, ECM components are degraded, mainly by endopeptidase type enzymes.

Lastly, the decrease in cellular defenses increases damage and disorders induced by external stresses such oxidative stress. The skin is then subjected to an aging process leading to the appearance of defects and blemishes of keratinous substances, in particular of the skin.

**Chitosan** is a linear polysaccharide composed of randomly distributed β-(1→4)-linked D-glucosamine and N-acetyl-D-glucosamine. Chitosan (CHI) is produced from the alkaline deacetylation of chitin. The ratio between D-glucosamine and N-acetyl-D-glucosamine units is considered as the degree of deacetylation (L. Bedian, A.M. Villalva-Rodriguez, G. Hernández-Vargas, R. Parra-Saldivar, H.M.N. Iqbal, Bio-based materials with novel characteristics for tissue engineering applications - a review, Int. J. Biol. Macromol. 98 (2017)837-846*).* When the deacetylation degree of CHI reaches circa 50%, it becomes soluble in aqueous acidic media [K.L.B. Chang, G. Tsai, J. Lee, W.R. Fu, Heterogeneous N-deacetylation of chitinin alkaline solution, Carbohydr. Res. 303 (1997) 327-332]. In acidic media the amino groups protonate, and the polymer becomes cationic, allowing it to interact with negatively charged molecules. This process is thought to be responsible for the antimicrobial activity of CHI, via the interaction of the polymer positive charges with the negatively charged cell membranes of microorganisms [P. Cazón, G. Velázquez, J.A. Ramirez, M. Vázquez, Polysaccharide-based films and coatings for food packaging: a review, Food Hydrocolloids 68 (2017) 136-148].

CHI is hydrophilic, permeation enhancer, mucoadhesive and a gelling agent. Besides, it is biocompatible, biodegradable, bioabsorbable and it does not produce inflammatory reactions (A. Muxika, A. Etxabide, J. Uranga, P. Guerrero, K. de la Caba, Chitosan as a bioactive polymer: Processing, properties and applications. International Journal of Biological Macromolecules 105 (2017) 1358-1368). Such properties make CHI an ideal polymer for the development of drug delivery systems. In addition, CHI displays further applications in biomedical, cosmeceutical and nutraceutical fields:
*Antimicrobial agent:* CHI exhibits free amino groups positively charged at pH below 6.3 which interact with the negative charges of the cell wall of the microorganisms and provoke the lysis (breakdown) of those structures. As a consequence, protein compounds and other intracellular constituents are lost and the microorganism die (E. I. Rabea, M. E. T. Badawy, C. V. Stevens, G. Smagghe, W. Steurbaut. Chitosan as antimicrobial agent: applications and mode of action. Biomacromolecules 4 (2003), 1457-1465). Moreover, chitosan inhibits the production of toxins by chelating metals present in the external structures of microorganisms (R. G. Cuero, G. Osuji, A. Washington. N-carboxymethylchitosan inhibition of aflatoxin production: role of zinc. Biotechnology Letters. 13 (1991), 441-444) and interact with microbial DNA inhibiting the synthesis of proteins and enzymes (N. R. Sudarshan, D. G. Hoover, D. Knorr. Antibacterial Action of Chitosan Food Biotechnology. 6 (1992), 257-272).

*Wound dressing:* CHI works on a charge-based mechanism of mucoadhesion which forms a mechanical barrier on the bleeding site. Moreover, CHI boosts the tissue growth by promoting the adhesion of proliferation of cells (tissue growth) and avoids wound infection due to its antibacterial properties (S. S. Biranje et al. Cytotoxicity and hemostatic activity of chitosan/carrageenan composite wound healing dressing for traumatic hemorrhage. Carbohydrate Polymers 239 (2020) 116106*)*.

*Tissue engineering:* CHI base scaffolds have been developed for cartilage and bond tissue regeneration. In that regard CHI scaffolds promote cell viability, cell proliferation and cell infiltration into scaffold architectures. In addition, CHI scaffolds are completely biodegradable in vivo conditions (A. Muxika, A. Etxabide, J. Uranga, P Guerrero, K. de la Caba. Chitosan as a bioactive polymer: Processing, properties andapplications. International Journal of Biological Macromolecules 105 (2017) 1358-1368*).*

*Cosmetics:* CHI is used as well in cosmetics and cosmeceuticals (I. Aranaz, et al. Cosmetics and Cosmeceutical Applications of Chitin, Chitosan and Their Derivatives Polymers 10 (2018) 213; doi:10.3390/polym10020213*).* In that regard CHI has been used in multifunctional sunscreen formulations with antibacterial properties (R. Morsy, S. S. Ali, M. EI-Shetehy.. Development of hydroxyapatite-chitosan gel sunscreen combating clinical multidrug-resistant bacteria, J. Mol. Struct.1143 (2017) 251-258) as well as encapsulating agent of depigmenting agents (C. Chaouat, et al. Green microparticles based on achitosan/lactobionic acid/linoleic acid association. Characterization and evaluation as a new carrier system for cosmetics, J. Microencapsulation 16 (2017) 1-11*).*

*Nutraceutical:* dietary protocols containing chitosan are used for the management of body weight (S. K. Yong, T. W. Wong. Chitosan for Body Weight Management. Marine Nutraceuticals: Prospects and Perspectives, 2013, p. 151)*.* Chitosan binds fat and helps fat excretion, consequently, fat adsorption is inhibited, and body weight loss is promoted. Chitosan attaches to bile salts as well, which interrupts the micellization of fat and the activation of the enzyme lipase leading to fat excretion and body weight loss. In addition, chitosan is difficult to digest in the gastrointestinal tract. In gastric medium, the amine moiety of chitosan protonates and the polymer hydrates and swells to form a viscous gel. Such a gel displays a high water-holding ability and induces satiety and satiation.

**Chitosan/hyaluronic acid particles** are formed by the combination of CHI and HA polymer chains. CHI/HA particles have been used as nanocarriers in drug delivery due to the ability to vehiculize hydrophilic active molecules (Y. Parajó et al. Hyaluronic acid/Chitosan nanoparticles as delivery vehicles for VEGF and PDGF-BB. Drug Delivery, 17 (2010) 596-604*)* as well as hydrophobic active molecules (A. Turcsányi, et al. Chitosan-modified hyaluronic acid-based nanosized drug carriers. Int. J. Biol. Macromol. 148 (2020) 218-225*).*

Chitosan/hyaluronic acid (HA) based micro and nanoparticles are prepared preferentially by polyelectrolyte (electrostatic) complexation. In this process, the electrostatic interaction between the protonated amines of the chitosan and the carboxylate anions of the hyaluronic acid promote the formation of the particle.

Electrostatic complexation proceeds through a gentle preparation processes performed under mild conditions and without the use of chemical reactions. In this regard electrostatic interactions can be used for the encapsulation of charged molecules (A. Gennari et al. The different ways to chitosan/hyaluronic acid nanoparticles: templated vs direct complexation. Influence of particle preparation on morphology, cell uptake and silencing efficiency. Beilstein J. Nanotechnol. 10 (2019) 2594-2608*).*

The use of CHI and HA polymers of similar size produces very stable polyelectrolyte complexes that intrinsically are difficult to reverse. In order to improve the reversibility of the process HA may be used in combination with a low molecular weight polyanion such as tripolyphosphate (TPP) (M. de la Fuente et al. Invest. Ophthalmol. Visual Sci. 49 (2008) 2016-2024*;* N. M. Zaki et al. Pharm. Res. 26 (2009) 1918-1930).

CHI/HA particles have been used for the delivery of biologically active molecules such as vitamins tocopherol (vitamin E) and cholecalciferol (vitamin D3) (A. Turcsányi et al. Chitosan-modified hyaluronic acid-based nanosized drug carriers. Int. J. Biol. Macromol. 148 (2020),218-225*),* anticancer drugs (N P Akentieva et al. Development of chitosan-hyaluronic acid nanoparticles and study of their physico-chemical properties for targeted delivery of anticancer drugs. IOP Conf. Ser.: Mater. Sci. Eng. 848 (2020) 012002) as well as RNA (X. Deng et al. Hyaluronic acid-chitosan nanoparticles for co-delivery of MiR-34a and doxorubicin in therapy against triple negative breast cancer. Biomaterials, 35(2014), 4333-4344) and DNA nucleic acids (H-D. Lu et al. Novel Hyaluronic Acid-Chitosan Nanoparticles as Non-Viral Gene Delivery Vectors Targeting Osteoarthritis. Int. J. Pharm. 420 (2011) 358-365).

Internalization of the CHI/HA particles in the cell proceeds via recognition of the hyaluronic acid chain by the CD44 protein receptor of the cell surface while chitosan allows both for the entrapment of the active molecule and for the endosomolytic activity that enables its liberation in the cytoplasm (J. M. Rios de la Rosa et al. The CD44-Mediated Uptake of Hyaluronic Acid-Based Carriers in Macrophages. Adv. Healthcare Mater. 6 (2017) 1601012; DOI: 10.1002/adhm.201601012*).*

**Phytocannabinoids** have pharmacological activity as they interact with the central nervous system (psychotropic). However, only some of them are psychoactive and cause an alteration of perception, mood and can cause addiction.

Phytocannabinoid derivatives have a wide variety of therapeutic applications that can be divided according to different medical conditions:
- Neurological and psychiatric disorders: multiple sclerosis, spasticity, epilepsy, Parkinson's disease, Alzheimer's disease, anxiety, depression, stress, bipolar disorders,
- Digestive and eating disorders: anorexia, loss of appetite, nausea, diabetes, Crohn's disease,
- Pain and inflammation: headache, migraine, fibromyalgia, inflammation, arthritis, cramps, spinal cord injury, muscle spasms, sprains and muscle injuries. Treatment of pain of peripheral origin both chronic and acute (contusions, postoperative),
- Inflammatory skin conditions: acne, psoriasis, atopic dermatitis and treatment of conflicting wounds (bedsores, diabetic foot),
- Others: cancer, insomnia, lupus, hypertension, ischemia, muscular dystrophy, fatigue, glaucoma, asthma.

Phytocannabinoids dissolve easily in lipids, alcohols and other non-polar organic solvents, but display very low water solubility. This fact limits its bioavailability and makes its encapsulation necessary in order to optimize its therapeutic activity and minimize its possible addictive properties.

Within the family of phytocannabinoids, the derivatives that have received the most attention are THC (tetrahydrocannabinol) that causes dependence, as well as CBD (cannabidiol) and CBG (cannabigerol) that are not addictive. In this sense, the US regulatory agency FDA (Food and Drug Administration) has approved several medications that contain THC and CBD derivatives in its composition: Marinol^{®} (dronabinol) and Cesamet^{®} (nabilone) that are synthetic analogs of tetrahydrocannabinol (THC) Epidiolex ^{®} which is a pure cannabidiol (CBD) extracted from the plant and Sativex^{®} (nabiximols): cannabis extract with a 1: 1 ratio of THC and CBD. Of these medicines only Sativex has been approved by the EMA (European Medicines Agency) for use in the EU.

**Cannabidiol (CBD)** is the non-psychoactive analog of tetrahydrocannabinol (THC). From a pharmacological point of view cannabidiol has little binding affinity for either CB1 and CB2 receptor but is capable of antagonizing them in the presence of other phytocannabionoids such as THC. CBD also regulates the perception of pain by affecting the activity of a significant number of targets including non-phytocannabinoid G protein-coupled receptors, ion channels and peroxisome proliferator- activated receptor. CBD displays as well anti-inflammatory and anti-spasmodic benefits. Other phytocannabinoids that can contribute to the analgesic effects of CBD is for instance cannabigerol (CBG). Similarly to CBD, CBG does not display significant affinities for phytocannabinoid receptors but they have other modes of action.

Phytocannabinoids, and, in particular, cannabidiol (CBD), display anti-inflammatory properties. Evidence exists that the effect of phytocannabinoids is the attenuation of the inflammatory component that occurs in acne vulgaris condition (A. Oláh et al. Cannabidiol exerts sebostatic and anti-inflammatory effects on human sebocytes. J Clin Invest. 124(2014)3713-3724*).* Moreover, the endocannabinoid system (ECS) regulates multiple physiological processes, including cutaneous cell growth and differentiation (A. Telek et al. Inhibition of human hair follicle growth by endo- and exocannabinoids. Faseb J. 21 (2007) 3534-3541)*.* In this regard, administration of CBD to cultured human sebocytes and human skin organ culture suppresses the lipogenic actions of arachidonic acid and of the combination of linoleic acid and testosterone and inhibits sebocyte proliferation through the activation of transient receptor potential vanilloid-4 (TRPV4) ion channels. Activation of TRPV4 interfers with the ERK1/2 MAPK pathway and provokes the downregulation of nuclear receptor interacting protein-1 (NRIP1), which affects glucose and lipid metabolism, and thus inhibits sebocyte lipogenesis. CBD also performs complex antiinflammatory actions coupled to A2a adenosine receptor-dependent upregulation of tribbles homolog 3 (TRIB3) and inhibition of the NF-κB signaling (A. Oláh et al. Cannabidiol exerts sebostatic and anti-inflammatory effects on human sebocytes. J Clin Invest. 124(2014)3713-3724*).* Therefore, CBD displays combined lipostatic, antiproliferative, and antiinflammatory effects that makes CBD a capable therapeutic agent for the treatment of acne vulgaris.

Other phytocannabinoids such as cannabigerol (CBG) and cannabigerovarin (CBGV) display pro-lipogenic and anti-inflammatory properties on sebocyte cell lines and may have potential in the treatment of dry-skin syndromes whereas cannabichromene (CBC), cannabidivarin (CBDV) and especially delta-9-tetrahydrocannabivarin (THCV) suppress arachidonic acid-induced seborrhea-mimicking lipogenesis and show remarkable anti-inflammatory actions on sebocyte cell lines that make them promising anti-acne agents (A. Oláh et al. Differential effectiveness of selected non-psychotropic phytocannabinoids on human sebocyte functions implicates the introduction in dry/seborrhoeic skin and acne treatment. Exp. Dermatol.25 (2016) 701-707*).*

Phytocannabinoids, and specially CBD, can be used for the treatment of eye disorders. Concerning this, CBD treatment reduces oxidative stress, decreases vascular hyperpermeability in the diabetic retina, protects retinal neurons activity and prevents retinal cell death (A. B. EI-Remessy et al. Neuroprotective and blood-retinal barrier preserving effects of cannabidiol in experimental diabetes. Am. J. Pathol. 168 (2006) 235-244*)*.

CBD affects both lipid and glucose metabolism through the action on various receptors as well as several metabolites. From the existing data it can be concluded that CBD might be effective in alleviating the symptoms of insulin resistance, type 2 diabetes and metabolic syndrome (P. Bielawiec. et al. Phytocannabinoids: Useful Drugs for the Treatment of Obesity? Special Focus on Cannabidiol. Front. Endocrinol. 11 (2020) art. 114; DOI: 10.3389/fendo.2020.00114*).*

Chitosan and hyaluronic acid have been used as stabilizers in the preparation of micellar suspensions that contain cannabinoids (WO2013009928, WO2015/068052). Patent WO2017/147691 refers to orally administrable pharmaceutical composition comprising a micellar composition that encapsulates cannabinoids and a film forming agent. Hyaluronic acid can be selected as a micelle forming compound whereas chitosan can be used as a film forming agent, which means that hyaluronic acid and chitosan do not interact with each other and therefore they do not form a particle. These documents refer to micelles formed from hyaluronic acid but not to chitosan/hyaluronic acid polymer particles or submicroparticles which are formed in the present invention and that contain phytocannabinoids.

Other patent document (WO2016154032) describes the use of containers comprising a permeable membrane and a polymeric matrix that contain cannabinoids for the preparation of beverages. Such a polymer matrix can comprise one or more components selected from chitosan and hyaluronic acid among others. This document patent however does not relate to the formation of chitosan/hyaluronic submicroparticles which are synthesized in the present invention and that contain phytocannabinoids.

Patent documents WO2013009928, WO2015/068052, WO2016154032 and WO2017/147691 may possess hyaluronic acid, chitosan and cannabinoids, however they do not refer to the formation of chitosan/hyaluronic acid particles nor chitosan/hyaluronic acid micro- and submicroparticles that are able to entrap cannabinoids in their structure.

Patent WO2018175637 refers to an invention to produce a dry powder that may contain hyaluronic acid and chitosan as polymer binding agents to afford micron size particles. Nevertheless, such particles are not intended for topical use and their size do not allow them to cross most biological barriers (D. S. Kohane, Microparticles and Nanoparticles for Drug Delivery, Biotechnology and Bioengineering, 96, 2007, 203-209*).* POTAS JOANNA ET AL: "Challenges in developing of chitosan - Based polyelectrolyte complexes as a platform for mucosal and skin drug delivery", EUROPEAN POLYMER JOURNAL, vol. 140, 1 November 2020, discloses CHI-based polyelectrolyte complexes with glycosaminoglycans such as hyaluronic acid, said complexes being in the form of nanoparticles used as carrier for drugs (e.g. indomethacin).

The above mentioned background corresponds to examples where phytocannabinoids are dissolved in organic solvents or with the aid of organic solvents, which are not suitable for systemic or topical applications in humans, or where cannabinoids are vehiculized in micellar systems where micelles are not formed with chitosan which is essential for the liberation of the cannabinoid in the cytoplasm via a endosomolytic process, or where cannabinoids are included in micron sized microparticles that do not cross most biological barriers.

The authors of the present invention, after an important experimental effort, have developed a new method for the solubilization of cannabinoids in aqueous media, preferably in the presence of sea water, for systemic and topical application via the formation of chitosan/hyaluronic acid submicroparticles which entrap cannabinoids and can cross biological barriers and subsequently are incorporated in the cells.

The effort of the authors of the present invention to perform the solubilization of the cannabinoids in aqueous media, through their incorporation in chitosan/hyaluronic acid submicroparticles, has led to obtain monophase gels, aqueous solutions and solid ingredients that allow the aqueous solubilization of cannabinoids to carry out relevant pharmaceutical, cosmetic and nutraceutical applications. Examples of such applications are the treatment of skin conditions, for instance acne, atopic dermatitis and psoriasis taking advance of the enhanced anti-inflammatory effect and lipogenic management capability for the phytocannabinoids combined with sea water, the treatment of eye disorders, such **as** diabetic retina, and the management of the body weight.

### Brief description of the drawings

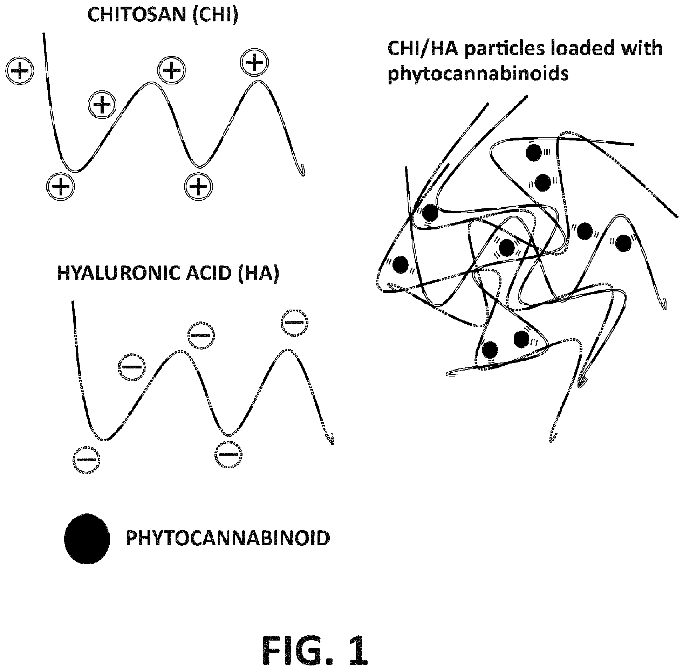

**Figure 1****. Configuration of the polyelectrolite complex.** Phytocannabinoids **(balls)** are placed in hydrophobic patches **(black regions)** formed between the protonated amine (+) of chitosan **(hollow regions)** and carboxylate anions (-) of hyaluronic acid **(dotted regions); (=)** interactions between pytocannabinoids and hydrophobic patches.

### Detailed description of the invention

In response to the needs of the state of the art, the authors of the invention have performed new methods for solubilization of cannabinoids in aqueous media through their incorporation in polymer submicroparticles, preferably in the presence of sea water. The subsequent product is suitable for pharmaceutical, cosmetic and nutraceutical applications.

In a first aspect, the present invention refers to a composition comprising cannabinoid loaded polymer submicroparticles soluble in aqueous media wherein said submicroparticles comprise:
- a polyelectrolyte complex formed by:
   o a positive charged polymer selected from chitosan
   o a negative charged polymer selected from hyaluronic acid or a salt thereof,
- a cannabinoid, or a mixture of cannabinoids, and
- water,
being the size of the submicroparticles from 600 to 750 nm, the weight ratio of positive charged polymer/negative charged polymer from 2.6:1 to 3.8:1, and the weight ratio polymers/cannabinoid from 100:1 to 1:100, preferably from 10:1 to 1:10 and most preferably from 5:1 to 1:5, and wherein the cannabinoids are arranged in hydrophobic patches formed between the positive charged polymer and the negative charged polymer chains.

In a particular embodiment, the pH of the composition ranges from 4 to 7.8, preferably from 4.5 to 5.2.

Submicroparticles are formed due to polyelectrolyte complexation. In this process, electrostatic interaction occurs between the protonated amines of the chitosan and the carboxylate anions of the hyaluronic acid and therefore promotes the formation of the particles (nucleation). The increase in the size of the particles proceeds via aggregation due to interaction of patches of the polymer chains of different charge (protonated amines of the chitosan and carboxylate anions of hyaluronic acid).

The internalization of the CHI/HA particles of the present invention in the cell proceeds via recognition of the hyaluronic acid chains by the CD44 protein receptor of the cell surface. Once molecular recognition occurs, vesicles containing the CHI/HA particles are formed. Such vesicles get entrapped into endosomes which later disrupt and liberate the cannabinoids in the cytoplasm due to the endosomolytic activity of the chitosan chains.

Cannabinoids are dibenzopyrane or benzochromen derivatives and in all cases hydrophobic molecules. The incorporation of the cannabinoids into the submicroparticles takes place in the hydrophobic patches formed between the chitosan and hyaluronic acid chains (see figure 1). CHI and HA possess a hydrophobic patch of eight or nine CH units, stretching along three neighboring sugar units and present on alternate sides of tapelike helices. Such helices interact between neighbouring CHI and HA chains and create hydrophobic pockets where cannabinoids accommodate.

In a preferred embodiment, cannabinoids are a phytocannabinoid or a mixture of phytocannabinoids.

Phytocannabinoids can be obtained not only from natural sources but also from chemical synthesis, biochemical synthesis or from genetically modified microorganism. Accordingly, phytocannabinoids used in the formulation of the present invention can be natural or synthetic.

Phytocannabinoids can be selected, among others, from cannabigerolic acid, cannabigerolic acid monomethylether, cannabigerol, cannabigerolmonomethylether, cannabigerovarinic acid, cannabigerovarin, cannabichromenic acid, cannabichromene, cannabichromevarinic acid, cannabichromevarin, cannabidiolic acid, cannabidiol, cannabidiol monomethylether, cannabidiol C4, cannabidivarinic acid, cannabidivarin, cannabidioreol, delta-9-(trans)-tetrahydrocannabinolic acid A, delta-9-(trans)-tetrahydrocannabinolic acid B, delta-9-(trans)-tetrahydrocannabinol, delta-9-(trans)-tetrahydrocannabinolic acid C4, delta-9-(trans)-tetrahydrocannabinol-C4, delta-9-(trans)-tetrahydrocannabivarinic acid, delta-9-(trans)-tetrahydrocannabivarin, delta-9-(trans)-tetrahydrocannabiorcolic acid, delta-9-(trans)-tetrahydrocannabiorcol, delta-8-(trans)-tetrahydrocannabinolic acid, delta-8-(trans)-tetrahydrocannabinol, cannabicyclolic acid, cannabicyclol, cannabicyclovarin, cannabielsoic acid A, cannabielsoic acid B, cannabielsoin, cannabinolic acid, cannabinol, cannabinol methylether, cannabinol-C4, cannabivarin, cannabiorcol, cannabinodiol, cannabinodivarin, (-)-cannabitriol, (+)-cannabitriol, (±)-9,10-dihydroxy-delta 6a(10a)-tetrahydrocannablnol, (-)-10-ethoxy-9-dihydroxy-delta 6a(10a)-tetrahydrocannabinol, (±)-8,9-dihydroxy-delta 6a(10a)-tetrahydrocannabinol,cannabidiolic acid tetrahydrocannabitriol ester and mixtures thereof.

In a particular embodiment, the phytocannabinoids are selected from cannabidiol, cannabigerol or a mixture thereof. In another particular embodiment a cannabis sativa extract can be used as phytocannabinoids source. The cannabis sativa extract comes from any part of the cannabis sativa plant including flower, leaf, stem and seeds.

The polymers used in the formulations of the present invention can be natural or synthetic polymers.

In a preferred embodiment the negative charged polymer is a hyaluronic acid salt of low molecular weight (M), where M ≤0.75·10⁶ Da, or a hyaluronic acid salt of high molecular weight (M), where M ≤2.2·10⁶ Da, or a mixture thereof, more preferably the hyaluronic acid salt is of low molecular weight, where 0.5·10⁶ Da≤ M ≤0.75·10⁶ Da or a hyaluronic acid salt of high molecular weight (M), where 1.9,10⁶ ≤ M ≤2.2·10⁶ Da or a mixture thereof. In a most preferred embodiment, the hyaluronic acid salt is of a molecular weight from 0.5 to 0.75 MDa.

Said low molecular or high molecular weight salts are of the same nature. In a most preferred embodiment, they hyaluronic acid salt is sodium hyaluronate.

In a preferred embodiment the positive charged polymer is chitosan of a molecular weight from 0.7 to 1MDa.

In a preferred embodiment the degree of deacetylation of chitosan is >20%, in a more preferred embodiment the degree of deacetylation is> 50% and most preferably the degree of acetylation is > 70%.

In a preferred embodiment, the water component of the composition of the invention is sea water. The incorporation of sea water in the formulation increases the cannabinoid encapsulation comparing to regular water.

Additionally, sea water enhances the benefits of cannabinoids for the treatment of skin conditions such as acne, atopic dermatitis and psoriasis and for the management of eye disorders.

In the composition of the invention, the size of the CHI and HA chains are similar in molecular weight to each other which, together with the presence of the cations of seawater that acts as a counterion of the excess of negative charges of HA with respect to the positive ones of CHI, results in the formation of submicroparticles with a homogeneous particle size distribution with a Polydispersity Index (PDI) from 0.25 to 0.9, preferably from 0.35 to 0.5.

In a second aspect, the present invention refers to a method of producing the composition comprising cannabinoid loaded polymer submicroparticles soluble in aqueous media, said method comprising the steps of:
a) Obtaining a solution of the positive charged polymer dissolved in an acidic aqueous solution, said solution consisting of acetic acid and sodium acetate, with pH adjusted at 4-4.5,
b) Obtaining a solution of the negative charged polymer dissolved in an acidic aqueous solution, said solution selected from a buffer, consisting of an acetic acid and sodium acetate, or water, with pH adjusted at 4-4.5,
c) Obtaining a solution of a cannabinoid, or a derivative thereof, or a mixture of cannabinoids, or derivatives thereof, by dissolving said cannabinoids in a solvent selected from ethanol, methanol and 1-isopropanol,
d) Addition of the solution obtained in c) to the solution obtained in a),
e) Addition of the solution obtained in b) to the solution obtained in d), and
f) Shaking and mechanical or magnetic stirring the solution obtained in e) until the solvent is evaporated.

The positive charged polymer is selected from chitosan or a derivative thereof and the negative charged polymer is selected from hyaluronic acid or a derivative thereof.

In a particular embodiment, the solution of step a) comprises chitosan in a concentration 2-6% (w/w), preferably 2.5-5% (w/w) with a molecular weight from 0.7 to 1 MDa. Preferably, the acidic aqueous solution consists of acetic acid 0.45% and sodium acetate 0.55% and the pH is adjusted with a suitable solution of HCl 5M and/or NaOH 5M.

In another particular embodiment, the solution of step b) comprises a hyaluronic acid salt, in a concentration from 0.5 to 3% (w/w), preferably 0.5-2.5% (w/w). Preferably, the hyaluronic acid salt is sodium hyaluronate of a molecular weight from 0.5 to 0.75 MDa.

In step b), the acidic aqueous solution is preferably selected from:
- a buffer consisting of acetic acid, in a concentration from 0.2% to 0.9%, and sodium acetate, in a concentration from 0.2% to 0.9%, or
- an aqueous solution consisting of sea water, in a concentration from 0.1 to 10%, preferable from 3 to 4% (w/v), with the pH adjusted by the addition of HCl.

In step c) the solution of cannabinoids is dissolved in a proper solvent, preferably selected from the group consisting of methanol, ethanol, 1-propanol and mixtures thereof. The concentration of the cannabinoid in the solvent (w/v) is 0.1-50%, preferably 10-40% and more preferably 15-30%.

In step d) the solution of cannabinoids is added to the solution of step a) in a ratio (v/v) 0.04:1-0.2:1, preferably 0.04:1-0.1:1.

Preferably, the cannabinoids are phytocannabinoids selected from cannabidiol, cannabigerol or a mixture thereof.

In step e) the solution obtained in step b) is added to solution obtained in step d) in a ratio (v/v) 0.5:1-1:0.5, preferably 0.7:1-1:0.7, more preferably 0.8:1-1:0.8.

In step f) the solution thus obtained is shaken for a period from 30 s to 5 min, preferably from 30 s to 90 s, followed by mechanical or magnetic stirring for a period from 12h to 48h, preferably from 12h to 36h, and more preferably from 18h to 30h.

The method provides homogeneously distributed submicroparticles that contain cannabinoids.

The methods of the present invention afford scalability so that the fabrication method can be performed at industrial level (manufacturing).

The compositions of the invention are suitable for pharmaceutical, cosmetic and nutraceutical applications.

Therefore, in another aspect, the invention refers to a pharmaceutical composition comprising the cannabinoid formulations of the present invention and their uses in different pharmaceutical or medical applications. In particular, the present invention refers to the use of this pharmaceutical composition in the treatment of skin conditions such as acne, atopic dermatitis and psoriasis and for the treatment of eye disorders.

The presence of sea water in the composition of the invention enhances the benefits of cannabinoids on the skin and also on eye disorders.

The present invention also refers to a cosmetic composition comprising the phytocannabinoid formulations of the present invention and its use for cosmetic applications. Specifically, the cosmetic composition of the invention affords compositions with relaxing, soothing and moisturizing effects on the skin.

The present invention also refers to a nutraceutical composition comprising the composition of the present invention and its use for nutraceutical applications. Specifically, nutraceutical compositions of the invention are useful for relaxation, calming and moisturization of the skin.

The composition of the invention can be formulated for topical, systemic or oral administration. For example, compositions can be formulated as a solid ingredient, topical gel/serum, eye/nasal drop and oral suspension.

Suitable compositions for topical administration can be formulated as gels, ointments, creams, lotions, drops, etc.

The systemic administration of the composition includes delivering the phytocannabinoid composition by injection, wherein the injection is intravenous, intra-articular, intramuscular, intradermal, intraspinal, intraperitoneal, subcutaneous, a bolus or a continuous administration. The composition of the invention can also be administered by intranasal administration.

The composition of the invention can also be administered by oral administration, such edible gels in the case of nutraceutical compositions.

The compositions of the invention can also be administered including in a medical device. Specifically, the composition of the invention can be drawn into a syringe for a water-based injection medium.

In particular embodiments, the resulting formulation can be liophilized in order to obtain a solid product that can be included as an ingredient in the formulation of pharmaceutical, cosmetic and nutraceutical product.

Submicroparticles can be also embedded in a matrix of hydroxypropylmethylcellulose (HPMC) in order to generate different final products, such as serum and eye drops.

Resulting formulations can be optionally sterilized, as in the case of injectable formulations. Sterilization process may be performed by steam sterilization, in an autoclave at a temperature ranging from 120°C to 140°C. In particular, the sterilization can be performed at 121° for 15 to 20 minutes, preferably 15 min, to obtain F0>15 (sterilizing value). Dry-heat is also employed to achieve sterilization.

### Examples

### Analytical Techniques:

### Viscosity

The viscosity of the product was obtained at 25°C using cone-and-plate geometry of 40 mm diameter and a truncation (gap) of 115 µm, at a shear rate of 1 s⁻¹. Formulations made from this product were measured at a shear rate of 1s⁻¹, except for the drops, which were measured using a shear rate of 66 s⁻¹.

### pH

pH of the product is determined after calibrating the pH meter with a minimum sensitivity of 95%.

### High Performance Liquid Cromatography (HPLC)

This technique was used to determine the total content of phytocannabinoids (CBD) in the formulations and the amount of CBD encapsulated in the chitosan:hyaluronic acid submicroparticles.

The analysis of the total amount of CBD in the system was performed by direct dilution of the samples in methanol followed by filtration through disposable 0.22 µm PVDF filters and subsequent injection in the chromatography equipment. In addition, for the determination of the encapsulated CBD, the product was filtered through disposable 0.22 µm PVDF filters, subsequently diluted in methanol and refiltered through these PVDF filters.

A HPLC-DAD analytical method according to **Table 1** was developed in order to quantify the CBD concentration in the formulations.

**Table 1 Chromatographic method for the quantification of CBD.**

| | |
|---|---|
| System | HPLC (1260 series Agilent Technologies) |
| Column | Zorbax Eclipse XCB-C18 (150×4.6 mm, 5 µm particle, Agilent) |
| Mobile-Phase | Channel A: Ammonium formate 10 mM (pH 3.6, with formic acid) |
| | Channel B: Acetonitrile |
| Gradient | 0-4 min 52-80% of B; 4-9.5 min 80 % of B at 1 ml/min (post-run= 2 min) |
| Detector | DAD (210, 228 and 270 nm) |

In this method a stock solution of 1000 mg/L of CBD in methanol was prepared from which 0.5, 1, 3, 5, 7.5, 10, 20 and 30 mg/L standard dilutions of CBD in methanol were made.

### Dynamic Light Scattering (DLS)

DLS measurements were performed by diluting 200 µL of the samples in 1000 µL of water and filtering them using PES syringe filters of 0.45 µm followed by analysis in the DLS equipment at 173° measurement angle. Attenuator value, measurement position and count number were employed as measurement quality indicators (table 2). In addition, zeta potential was measured at 40 V voltage.

**Table 2: DLS analysis method parameters for the characterization of vehiculized CBD.**

| | |
|---|---|
| Dispersant | Water |
| Temperature | 25°C |
| Viscosity | 0.8872 cP |
| RI | 1.330 |
| Dielectric constant | 78.5 |
| Fitting model | Smoluchowski |
| Equilibration time | 120 s |
| Cell time | Capillary cell DTS1070 |
| Measurement angle | 173° |
| Number of measurements per sample | 3 |

### PREPARATION OF ARTIFICIAL -SEA WATER (SW)

Artificial Sea Water was prepared as follows:
1 L of artificial SW was prepared by dissolving Sodium chloride (24.53g), Magnesium chloride (5.20g), Sodium sulfate (4.09g), Calcium chloride (1.16g), Potassium chloride (0.695g), Sodium bicarbonate (0.201g), Potassium Bromide (0.101g), Boric acid (0.027g), Strontium chloride (0.0025g) and Sodium Fluoride (0.003g) in purified water (988.968g). The solution was kept under magnetic stirring until the complete dissolution of the reactants was reached. Artificial Sea Water was prepared according to ASTM D1141-1 98.

### EXAMPLE 1. CHI:HA-CBD submicroparticle solution without sea water (CHI:HA - 1.25:0.38 with 1.3 mg CBD)

0.5 g of Chitosan (MW 0.70 MDa) was dissolved in 20 mL acetic acid and sodium acetate buffer (pH 4.22) for 12 hours to obtain a chitosan solution of 2.5% (v/w). The pH of the solution was adjusted to 4.5 by the addition of appropriate volumes of HCl 5 M. 0.150 g of low molecular weight hyaluronic acid (MW 0.66 MDa) was added via spatula to 20 mL acetic acid and sodium acetate buffer (pH 4.22). The resulting solution was stirred mechanically for 12h until complete solution of the polymer was reached. Following a solution of phytocannabinoid (1.3 mg of CBD) in 130 microL of ethanol absolute was added to 2 mL of the 2.5% chitosan solution. 2 mL of the previously prepared 0.75% (w/v) HA solution were added to the CHI and CBD solution while it was kept under magnetic stirring. The resulting solution was shaken for 60 seconds followed by mechanical stirring for 30 minutes. Finally, the solution was kept under magnetic stirring for 24 hours to ensure evaporation of ethanol.

### EXAMPLE 2. CHI:HA-CBD submicroparticle solution (CHI:HA - 1.25:0.38 with 1.3 mg CBD)

The experiment of example 1 was repeated using sea water for the solubilization of hyaluronic acid instead of using acetic acid/sodium acetate buffer. The protocol was adapted as follows:
0.5 g of Chitosan (MW 0.70 MDa) was dissolved in 20 mL acetic acid and sodium acetate buffer (pH 4.39) for 12 hours to obtain a chitosan solution of 2.5% (v/w). The pH of the solution was adjusted to 4.5 by the addition of appropriate volumes of HCl 5 M. 0.150 g of low molecular weight hyaluronic acid (MW 0.66 MDa) was added via spatula to 20 mL of sea water (pH 4.03, adjusted with HCl). The resulting solution was stirred mechanically for 12h until complete solution of the polymer was reached. Following a solution of phytocannabinoid (1.3 mg of CBD) in 130microL of ethanol absolute was added to 2 mL of the 2.5% chitosan solution. 2 mL of the previously prepared 0.75% (w/v) HA solution were added to the CHI and CBD solution while it was kept under magnetic stirring. The resulting solution was shaken for 60 seconds followed by mechanical stirring for 30 minutes. Finally, the solution was kept under magnetic stirring for 24 hours to ensure evaporation of ethanol.

### EXAMPLE 3. CHI:HA-CBD submicroparticle solution (CHI:HA - 1.25:0.38 with 20 mg CBD)

The experiment of example 2 was repeated increasing the CBD% of the formulation from 0.032% (w/v) to 0.5% (w/v). The protocol was adapted as follows:
0.5 g of Chitosan (MW 0.70 MDa) was dissolved in 20 mL acetic acid and sodium acetate buffer (pH 4.39) for 12 hours to obtain a chitosan solution of 2.5% (v/w). The pH of the solution was adjusted to 4.5 by the addition of appropriate volumes of HCl 5 M. 0.150 g of low molecular weight hyaluronic acid (MW 0.66 MDa) was added via spatula to 20 mL of sea water (pH 4.03 (adjusted with HCl)). The resulting solution was stirred mechanically for 12h until complete solution of the polymer was reached. Following a solution of phytocannabinoid (20 mg of CBD) in 92.6 microL of ethanol absolute was added to 2 mL of the 2.5% chitosan solution. 2 mL of the previously prepared 0.75% (w/v) HA solution were added to the CHI and CBD solution while it was kept under magnetic stirring. The resulting solution was shaken for 60 seconds followed by mechanical stirring for 30 minutes. Finally, the solution was kept under magnetic stirring for 24 hours to ensure evaporation of ethanol.

### EXAMPLE 4. CHI:HA-CBD submicroparticle solution without sea water (CHI:HA - 2.5:0.75, no sea water)

The experiment of example 1 was repeated doubling the polymer concentration and increasing the CBD concentration from 0.032% (w/v) to 0.98% (w/v). The protocol was adapted as follows:
1 g of Chitosan (MW 0.70 MDa) was dissolved in 20 mL acetic acid and sodium acetate buffer (pH 4.39) for 12 hours to obtain a chitosan solution of 5% (v/w). The pH of the solution was adjusted to 4.5 by the addition of appropriate volumes of HCl 5 M. 0.3 g of low molecular weight hyaluronic acid (MW 0.66 MDa) was added via spatula to 20 mL of acetic acid and sodium acetate buffer (pH 4.39). The resulting solution was stirred mechanically for 12h until complete solution of the polymer was reached. Following, a solution of phytocannabinoid (39 mg of CBD) in 177 microL of ethanol absolute was added to 2 mL of the 5% chitosan solution. 2 mL of the previously prepared 1.5% (w/v) HA solution were added to the CHI and CBD solution while it was kept under magnetic stirring. The resulting solution was shaken for 60 seconds followed by mechanical stirring for 30 minutes. Finally, the solution was kept under magnetic stirring for 24 hours to ensure evaporation of ethanol.

### EXAMPLE 5. CHI:HA-CBD submicroparticle solution (CHI:HA - 2.5:0.75)

The experiment of example 4 was repeated using sea water for the solubilization of hyaluronic acid instead of acetic acid/sodium acetate buffer. The protocol was adapted as follows:
1 g of Chitosan (MW 0.70 MDa) was dissolved in 20 mL acetic acid and sodium acetate buffer (pH 4.39) for 12 hours to obtain a chitosan solution of 5% (v/w). The pH of the solution was adjusted to 4.5 by the addition of appropriate volumes of HCl 5 M. 0.3 g of low molecular weight hyaluronic acid (MW 0.66 MDa) was added via spatula to 20 mL of sea water (pH 4.03 (adjusted with HCl)). The resulting solution was stirred mechanically for 12h until complete solution of the polymer was reached. The pH of the solution was adjusted to 4.5 by the addition of appropriate volumes of HCl 5 M. Following, a solution of phytocannabinoid (39 mg of CBD) in 177 microL of ethanol absolute was added to 2 mL of the 5% chitosan solution. 2 mL of the previously prepared 1.5% (w/v) HA solution were added to the CHI and CBD solution while it was kept under magnetic stirring. The resulting solution was shaken for 60 seconds followed by mechanical stirring for 30 minutes. Finally, the solution was kept under magnetic stirring for 24 hours to ensure evaporation of ethanol.

### EXAMPLE 6. CHI:HA-CBD submicroparticle solution (CHI:HA - 2.5:0.25)

The experiment of example 5 was repeated using a 0.5% (w/v) HA solution instead of 1.5% (w/v). The protocol was adapted as follows:
1 g of Chitosan (MW 0.70 MDa) was dissolved in 20 mL acetic acid and sodium acetate buffer (pH 4.39) for 12 hours to obtain a chitosan solution of 5% (v/w). The pH of the solution was adjusted to 4.5 by the addition of appropriate volumes of HCl 5 M. 0.1 g of low molecular weight hyaluronic acid (MW 0.66 MDa) was added via spatula to 20 mL of sea water (pH 4.4 (adjusted with HCl)). The resulting solution was stirred mechanically for 12h until complete solution of the polymer was reached. The pH of the solution was adjusted to 4.5 by the addition of appropriate volumes of HCl 5 M. Following, a solution of phytocannabinoid (39 mg of CBD) in 177microL of ethanol absolute was added to 2 mL of the 5% chitosan solution. 2 mL of the previously prepared 0.5% (w/v) HA solution were added to the CHI and CBD solution while it was kept under magnetic stirring. The resulting solution was shaken for 60 seconds followed by mechanical stirring for 30 minutes. Finally, the solution was kept under magnetic stirring for 24 hours to ensure evaporation of ethanol.

### EXAMPLE 7. CHI:HA-CBD submicroparticle solution (CHI:HA - 2.5:0.5)

The experiment of example 5 was repeated using a 1% (w/v) HA solution instead of 1.5% (w/v). The protocol was adapted as follows:
1 g of Chitosan (MW 0.70 MDa) was dissolved in 20 mL acetic acid and sodium acetate buffer (pH 4.39) for 12 hours to obtain a chitosan solution of 5% (v/w). The pH of the solution was adjusted to 4.5 by the addition of appropriate volumes of HCl 5 M. 0.2 g of low molecular weight hyaluronic acid (MW 0.66 MDa) was added via spatula to 20 mL of sea water (pH 4.54 (adjusted with HCl)). The resulting solution was stirred mechanically for 12h until complete solution of the polymer was reached. The pH of the solution was adjusted to 4.5 by the addition of appropriate volumes of HCl 5 M. Following, a solution of phytocannabinoid (39 mg of CBD) in 177 microL of ethanol absolute was added to 2 mL of the 5% chitosan solution. 2 mL of the previously prepared 1% (w/v) HA solution were added to the CHI and CBD solution while it was kept under magnetic stirring. The resulting solution was shaken for 60 seconds followed by mechanical stirring for 30 minutes. Finally, the solution was kept under magnetic stirring for 24 hours to ensure evaporation of ethanol.

### EXAMPLE 8. CHI:HA-CBD submicroparticle solution (CHI:HA - 2.5:1)

The experiment of example 5 was repeated using a 2% (w/v) HA solution instead of 1.5% (w/v). The protocol was adapted as follows:
1 g of Chitosan (MW 0.70 MDa) was dissolved in 20 mL acetic acid and sodium acetate buffer (pH 4.39) for 12 hours to obtain a chitosan solution of 5% (v/w). The pH of the solution was adjusted to 4.5 by the addition of appropriate volumes of HCl 5 M. 0.4 g of low molecular weight hyaluronic acid (MW 0.66 MDa) was added via spatula to 20 mL of sea water (pH 4.31 (adjusted with HCl)). The resulting solution was stirred mechanically for 12h until complete solution of the polymer was reached. The pH of the solution was adjusted to 4.5 by the addition of appropriate volumes of HCl 5 M. Following, a solution of phytocannabinoid (39 mg of CBD) in 177 microL of ethanol absolute was added to 2 mL of the 5% chitosan solution. 2 mL of the previously prepared 2% (w/v) HA solution were added to the CHI and CBD solution while it was kept under magnetic stirring. The resulting solution was shaken for 60 seconds followed by mechanical stirring for 30 minutes. Finally, the solution was kept under magnetic stirring for 24 hours to ensure evaporation of ethanol.

### EXAMPLE 9. CHI:HA-CBD submicroparticle solution (CHI: HA - 2.5:1.25)

The experiment of example 5 was repeated using a 2.5% (w/v) HA solution instead of 1.5% (w/v). The protocol was adapted as follows:
1 g of Chitosan (MW 0.70 MDa) was dissolved in 20 mL acetic acid and sodium acetate buffer (pH 4.39) for 12 hours to obtain a chitosan solution of 5% (v/w). The pH of the solution was adjusted to 4.5 by the addition of appropriate volumes of HCl 5 M. 0.5 g of low molecular weight hyaluronic acid (MW 0.66 MDa) was added via spatula to 20 mL of sea water (pH 4 (adjusted with HCl)). The resulting solution was stirred mechanically for 12h until complete solution of the polymer was reached. The pH of the solution was adjusted to 4.5 by the addition of appropriate volumes of HCl 5 M. Following, a solution of phytocannabinoid (39 mg of CBD) in 177 microL of ethanol absolute was added to 2 mL of the 5% chitosan solution. 2 mL of the previously prepared 2.5% (w/v) HA solution were added to the CHI and CBD solution while it was kept under magnetic stirring. The resulting solution was shaken for 60 seconds followed by mechanical stirring for 30 minutes. Finally, the solution was kept under magnetic stirring for 24 hours to ensure evaporation of ethanol.

### EXAMPLE 10. Solid ingredient

The solution resulting from the experiment 3 was lyophilized in order to obtain submicroparticles of CHI:HA loaded with CBD as a solid ingredient. 0.0962 g of the solid ingredient was reconstituted in 1mL of type II water and the pH, particle size, zeta potential, PDI and CBD loading (total and encapsulated) were analyzed.

### EXAMPLE 11. Topical gel/serum

The solution resulting from example 5 was modified by adding some ingredients in order to obtain a cosmetic product (serum) containing CBD loaded CHI:HA submicroparticles. The serum was prepared as follows:
0.5 g of glycerin (5% w/v) and 0.2 g of propyleneglycol (2% w/v) were added to 4 mL of the NP solution resulting from example 5 and it was kept under magnetic stirring for 10 minutes. 0.07 g (0.7% w/v) of high molecular weight hydroxypropylmethylcellulose (HPMC) (Benecel K100M) was added via spatula to the previous solution under magnetic stirring. Finally type II water was added (q.s. 10 mL) and the resulting mixture was shaken mechanically until the complete dissolution of the reactants was reached.

### EXAMPLE 12. Eye drops/nasal drops

The solution resulting from example 5 was modified in order to obtain an eye drop/nasal drop formulation containing CBD loaded CHI:HA submicroparticles. The eye or nasal drops were prepared as follows:
0.04 g (0.2% w/v) of high molecular weight hydroxypropylmethylcellulose (HPMC) (Benecel K100M) was added to 8 mL of the submicroparticle solution resulting from example 5 and it was kept under magnetic stirring. Then type II water was added (q.s. 20 mL) and the resulting mixture was shaken mechanically until the complete dissolution of the polymer was reached.

### EXAMPLE 13. Oral suspension

The solution resulting from example 5 was modified in order to obtain an oral suspension formulation containing CBD loaded CHI:HA submicroparticles. The oral suspension was prepared as follows:
0.2 g of glycerin (2% w/v) was added to 4 mL of the submicroparticle solution resulting from example 5 and it was kept under magnetic stirring for 10 minutes. 0.015 g of potassium sorbate (0.15% w/v), 0.2g of xylitol (2% w/v) and 0.2g of mannitol (2% w/v) were added via spatula to the previous solution under magnetic stirring and it was kept in agitation until the complete dissolution of the ingredients. Finally, 0.02 g (0.2% w/v) of xanthan gum and purified water (q.s 10 mL) were added to the previous solution and the resulting mixture was shaken mechanically until the complete dissolution of the reactants was reached.

### EXAMPLE 14.

**Table 3. Physicochemical characterization of CHI:HA-CBD NP formulations**

| Composition | Ratio CHI:HA | Total Cannabinoid (encapsulated + non-encapsulated) % (HPLC) | Encapsulated Cannabinoid % (HPLC) | Method of synthesis | pH | Size of particles (nm) | PDI |
|---|---|---|---|---|---|---|---|
| Example 1 NO SW | 1.25:0.38 | CBD 0.026% | 0.023% | Method example 1 | 4.37 | 638,64 | 0,29 |
| Example 2 | 1.25:0.38 | CBD 0.033% | 0.029% | Method example 2 | 4.52 | 692,56 | 0,35 |
| Example 3 | 1.25:0.38 | CBD 0.21% | 0.11% | Method example 3 | 4.61 | 629,36 | 0,35 |
| Example 4 NO SW | 2.5:0.75 | CBD 0.74% | 0.40% | Method example 4 | 4.41 | 734,17 | 0,86 |
| Example 5 | 2.5:0.75 | CBD 0.74% | 0.57% | Method example 5 | 5.19 | 613,27 | 0,50 |
| Example 6 | 2.5:0,2 | CBD 0.034% | 0.017% | Method example 6 | 4,15 | Aggregates | 0.97 |
| Example 7 | 2.5:0,5 | CBD 0.041% | 0.037% | Method example 7 | 4,15 | Aggregates | 1.00 |
| Example 8 | 2.5:1 | CBD 0.075% | 0.062% | Method example 8 | 4,06 | Aggregates | 1.00 |
| Example 9 | 2.5:1,25 | CBD 0.059% | 0.041% | Method example 9 | 4,11 | Aggregates | 1.00 |

### EXAMPLE 15.

**Table 4. Physicochemical characterization of final products containing CBD loaded CHI:HA submicroparticles**

| Composition | Total Cannabinoid (encapsulated + non-encapsulated) % (HPLC) | Encapsulated Cannabinoid % (HPLC) | Method of synthesis | pH | Size of particles (nm) | ^{η}(cP) |
|---|---|---|---|---|---|---|
| Example 10 | CBD 0.73% | 0.30% | Method example 10 | 5.48 | 327,97 | NA |
| Example 11 | CBD 0.10% | 0.040% | Method example 11 | 5.11 | 536,32 | 2585 |
| Example 12 | CBD 0.18% | 0.063% | Method example 12 | 4.99 | 1209,63 | 40 |
| Example 13 | CBD 0.15% | 0.090% | Method example 13 | 5.23 | 577,20 | NA |

Data from examples 14 and 15 show that cannabinoids are incorporated into CHI:HA particles formulated in aqueous media with and without sea water.

Incorporating sea water into the formulation produces an increase of the amount of encapsulated cannabinoids (example 2 vs example 1; example 5 vs example 4).

The ratios CHI:HA 2.5:0.75 and 1.25:0.38 (examples 1-5) are optimal for the formation of submicroparticles. When ratios out of this range were used, aggregates were formed and no submicroparticles were obtained, as shown in examples 6, 7, 8 and 9.

The ratio 2.5:0.75 maximizes the amount of total cannabinoid found in the formulation.

## Claims

1. Composition comprising cannabinoid loaded polymer submicroparticles soluble in aqueous media **characterized in that** the submicroparticles comprise:
- a polyelectrolyte complex formed by:
• a positive charged polymer selected from chitosan,
• a negative charged polymer selected from hyaluronic acid or a salt thereof, and
- a cannabinoid, or a mixture of cannabinoids, and
- water,
wherein the size of the submicroparticles ranges from 600 to 750 nm, the weight ratio of positive charged polymer/negative charged polymer is 2.6:1-3.8:1, and the weight ratio polymers/cannabinoid is 100:1 to 1:100, and wherein the cannabinoids are arranged in hydrophobic patches formed between the positive charged polymer and the negative charged polymer chains.

2. Composition according to claim 1 wherein the pH of the composition ranges from 4 to 7.8, preferably from 4.5 to 5.2.

3. Composition according to claim 1 or 2 wherein the weight ratio polymers/cannabinoid is from 10:1 to 1:10, preferably from 5:1 to 1:5.

4. Composition according to any of previous claims wherein the cannabinoid is a phytocannabinoid, or a mixture of phytocannabinoids, preferably selected from cannabidiol, cannabigerol or a mixture thereof.

5. Composition according to any of previous claims wherein the negative charged polymer is a hyaluronic acid salt of a molecular weight from 0.5 to 0.75 MDa, preferably sodium hyaluronate.

6. Composition according to any of previous claims wherein the positive charged polymer is chitosan of a molecular weight from 0.7 to 1 MDa.

7. Composition according to any of previous claims wherein the water is sea water.

8. Composition according to any of previous claims wherein the submicroparticles has a Polydispersity Index (PDI) from 0.25 to 0.9, preferably from 0.35 to 0.5.

9. A method of producing a composition comprising cannabinoid loaded polymer submicroparticles soluble in aqueous media, according to claims 1-8, **characterized in that** said method comprises the steps of:
a) Obtaining a solution of the positive charged polymer dissolved in an acidic aqueous solution, said solution consisting of acetic acid and sodium acetate, with pH adjusted at 4-4.5,
b) Obtaining a solution of the negative charged polymer dissolved in an acidic aqueous solution, said solution selected from a buffer, consisting of an acetic acid and sodium acetate, or water, with pH adjusted at 4-4.5,
c) Obtaining a solution of a cannabinoid, or a mixture of cannabinoids, by dissolving said cannabinoids in a solvent selected from ethanol, methanol and 1-isopropanol,
d) Addition of the solution obtained in c) to the solution obtained in a),
e) Addition of the solution obtained in b) to the solution obtained in d), and
f) Shaking and mechanical or magnetic stirring the solution obtained in e) until the solvent is evaporated.

10. Method according to claim 9 wherein, in step a), the solution comprises chitosan in a concentration from 2 to 6% (w/w), preferably from 2.5 to 5% (w/w), the acidic aqueous solution consists of acetic acid 0.45% (w/v) and sodium acetate 0.55% (w/v) and the pH is adjusted with a suitable solution of HCl 5M and/or NaOH 5M.

11. Method according to claim 9 or 10 wherein solution of step b) comprises a hyaluronic acid salt in a concentration from 0.5 to 3% (w/w), preferably from 0.5% to 2.5% (w/w).

12. Method according to any of claims 9-11 wherein the acidic aqueous solution of step b) is selected from
- a buffer consisting of acetic acid, in a concentration from 0.2% to 0.9%, and sodium acetate (w/v), in a concentration from 0.2% to 0.9% (w/v), or
- an aqueous solution consisting of sea water, wherein the concentration of salts in water is from 0.1 to 10% (w/v), preferable from 3 to 4% (w/v), with the pH adjusted by the addition of HCl.

13. Method according to any of claims 9-12 wherein in step c), the concentration of the cannabinoid in the solvent (w/v) is from 0.1 to 50%, preferably from 10 to 40% and more preferably from 15 to 30%.

14. Method according to any of claims 9-13 wherein in step d) the solution of cannabinoids is added to the solution of step a) in a ratio (v/v) from 0.04:1 to 0.2:1, preferably from 0.04:1 to 0.1:1.

15. Method according to any of claims 9-14 wherein in step e) the solution obtained in step b) is added to solution obtained in step d) in a ratio (v/v) from 0.5:1 to 1:0.5, preferably from 0.7:1 to 1:0.7, more preferably from 0.8:1 to 1:0.8.

16. Method according to any of claims 9-15 wherein in step f) the solution is shaken for a period from 30 s to 5 min, preferably from 30 s to 90 s, followed by mechanical or magnetic stirring for a period from 12 to 48 h, preferably from 12 to 36, and more preferably from 18 to 30.

17. A nutraceutical, pharmaceutical or cosmetic composition comprising the composition of any of claims 1-8.

18. Pharmaceutical composition according to claim 17 for use in the treatment of skin conditions selected from acne and atopic dermatitis.

19. Pharmaceutical composition according to claim 17 for use in the treatment of eye disorders selected from diabetic retina.

20. A non-therapeutic use of the cosmetic composition, according to claim 17, for relaxing, soothing and moisturizing effects on the skin.

## Patentansprüche

1. Zusammensetzung, umfassend Cannabinoid-beladene, in wässrigen Medien lösliche Polymer-Submikropartikel, **dadurch gekennzeichnet, dass** die Submikropartikel Folgendes umfassen:
- einen Polyelektrolytkomplex, gebildet durch:
• ein positiv geladenes Polymer, ausgewählt aus Chitosan,
• ein negativ geladenes Polymer, ausgewählt aus Hyaluronsäure oder einem Salz davon, und
- ein Cannabinoid oder eine Mischung aus Cannabinoiden und
- Wasser,
wobei die Größe der Submikropartikel im Bereich von 600 bis 750 nm liegt, das Gewichtsverhältnis von positiv geladenem Polymer zu negativ geladenem Polymer 2.6:1 - 3.8:1 beträgt und das Gewichtsverhältnis Polymeren/Cannabinoid 100:1 bis 1:100 beträgt, und wobei die Cannabinoide in hydrophoben Bereichen angeordnet sind, die zwischen dem positiv geladenen Polymer und dem negativ geladenen Polymer gebildet sind.

2. Zusammensetzung nach Anspruch 1, wobei der pH-Wert der Zusammensetzung im Bereich von 4 bis 7.8, bevorzugt von 4.5 bis 5.2 liegt.

3. Zusammensetzung nach Anspruch 1 oder 2, wobei das Gewichtsverhältnis Polymere/Cannabinoid 10:1 bis 1:10 beträgt, bevorzugt von 5:1 bis 1:5.

4. Zusammensetzung nach einem der vorherigen Ansprüche, wobei das Cannabinoid ein Phytocannabinoid oder eine Mischung aus Phytocannabinoiden ist, bevorzugt ausgewählt aus Cannabidiol, Cannabigerol oder einer Mischung davon.

5. Zusammensetzung nach einem der vorherigen Ansprüche, wobei das negativ geladene Polymer ein Hyaluronsäuresalz mit einem Molekulargewicht von 0.5 bis 0.75 MDa ist, bevorzugt Natriumhyaluronat.

6. Zusammensetzung nach einem der vorherigen Ansprüche, wobei das positiv geladene Polymer Chitosan mit einem Molekulargewicht von 0.7 bis 1 MDa ist.

7. Zusammensetzung nach einem der vorherigen Ansprüche, wobei das Wasser Meerwasser ist.

8. Zusammensetzung nach einem der vorherigen Ansprüche, wobei die Submikropartikel einen Polydispersitätsindex (POI) von 0.25 bis 0.9, bevorzugt von 0.35 bis 0.5, haben.

9. Verfahren zur Herstellung einer Zusammensetzung, umfassend in wässrigen Medien lösliche, Cannabinoid-beladene Polymer-Submikropartikel, gemäß den Ansprüchen 1 - 8, **dadurch gekennzeichnet, dass** das Verfahren die folgenden Schritte umfasst:
a) Bereitstellen einer Lösung des positiv geladenen Polymers, gelöst in einer sauren wässrigen Lösung, wobei besagte Lösung aus Essigsäure und Natriumacetat besteht, mit pH-Wert eingestellt auf 4 - 4.5,
b) Bereitstellen einer Lösung des negativ geladenen Polymers, gelöst in einer sauren wässrigen Lösung, wobei die Lösung ausgewählt ist aus einem Puffer, bestehend aus Essigsäure und Natriumacetat, oder Wasser, mit pH-Wert eingestellt auf 4 - 4.5,
c) Bereitstellen einer Lösung eines Cannabinoids oder einer Mischung von Cannabinoiden durch Auflösen besagter Cannabinoide in einem Lösungsmittel, ausgewählt aus Ethanol, Methanol und 1-Isopropanol,
d) Zugabe der in c) bereitgestellten Lösung zu der in a) bereitgestellten Lösung,
e) Zugabe der in b) bereitgestellten Lösung zu der in d) bereitgestellten Lösung, und
f) Schütteln und mechanisches oder magnetisches Rühren der in e) bereitgestellten Lösung, bis das Lösungsmittel verdampft ist.

10. Verfahren nach Anspruch 9, wobei in Schritt a) die Lösung Chitosan in einer Konzentration von 2 bis 6 % (Gew./Gew.), bevorzugt von 2.5 bis 5 % (Gew./Gew.), umfasst, die saure wässrige Lösung aus 0.45 % (Gew./Vol.) Essigsäure und 0.55 % (Gew./Vol.) besteht und der pH-Wert mit einer geeigneten Lösung von HCl 5M und/oder NaOH 5M eingestellt wird.

11. Verfahren nach Anspruch 9 oder 10, wobei die Lösung aus Schritt b) ein Hyaluronsäuresalz in einer Konzentration von 0.5 bis 3 % (Gew./Gew.), bevorzugt von 0.5 % bis 2.5 % (Gew./Gew.), umfasst.

12. Verfahren nach einem der Ansprüche 9 - 11, wobei die saure wässrige Lösung aus Schritt b) ausgewählt ist aus
- einem Puffer, bestehend aus Essigsäure in einer Konzentration von 0.2 % bis 0.9 % und Natriumacetat (Gew./Vol.), in einer Konzentration von 0.2 % bis 0.9 % (Gew./Vol.), oder
- einer wässrigen Lösung, bestehend aus Meerwasser, wobei die Konzentration der Salze im Wasser 0.1 bis 10 % (Gew./Vol.), bevorzugt 3 bis 4 % (Gew./Vol.) beträgt, und der pH-Wert durch Zugabe von HCl eingestellt wird.

13. Verfahren nach einem der Ansprüche 9 - 12, wobei in Schritt c) die Konzentration des Cannabinoids in dem Lösungsmittel (Gew./Vol.) 0.1 bis 50 %, bevorzugt 10 bis 40 % und noch bevorzugter 15 bis 30 % beträgt.

14. Verfahren nach einem der Ansprüche 9 - 13, wobei in Schritt d) die Lösung von Cannabinoiden zu der Lösung aus Schritt a) in einem Verhältnis (Vol./Vol.) von 0.04:1 bis 0.2:1, bevorzugt von 0.04:1 bis 0.1:1, zugegeben wird.

15. Verfahren nach einem der Ansprüche 9 bis 14, wobei in Schritt e) die in Schritt b) erhaltene Lösung zu der in Schritt d) erhaltenen Lösung in einem Verhältnis (Vol./Vol.) von 0.5:1 bis 1:0.5, bevorzugt von 0.7:1 bis 1:0.7, noch bevorzugter von 0.8:1 bis 1:0.8 zugegeben wird.

16. Verfahren nach einem der Ansprüche 9 bis 15, wobei in Schritt f) die Lösung für einen Zeitraum von 30 s bis 5 min, bevorzugt von 30 s bis 90 s, geschüttelt wird, gefolgt von einem mechanischen oder magnetischen Rühren für einen Zeitraum von 12 bis 48 h, bevorzugt von 12 bis 36 h und noch bevorzugter von 18 bis 30 h.

17. Nutrazeutische, pharmazeutische oder kosmetische Zusammensetzung, umfassend die Zusammensetzung nach einem der Ansprüche 1 - 8.

18. Pharmazeutische Zusammensetzung gemäß Anspruch 17 zur Verwendung bei der Behandlung von Hauterkrankungen, ausgewählt aus Akne und atopischer Dermatitis.

19. Pharmazeutische Zusammensetzung gemäß Anspruch 17 zur Verwendung bei der Behandlung von Augenerkrankungen, ausgewählt aus diabetischer Retinopathie.

20. Nicht-therapeutische Verwendung der kosmetischen Zusammensetzung gemäß Anspruch 17 zur Entspannung, Beruhigung und Befeuchtung der Haut.

## Revendications

1. Composition comprenant des submicroparticules de polymère chargées en cannabinoïde et solubles en milieu aqueux, **caractérisée en ce que** les submicroparticules comprennent :
- un complexe polyélectrolyte formé par :
• un polymère chargé positivement sélectionné à partir du chitosane,
• un polymère chargé négativement sélectionné parmi l'acide hyaluronique ou un sel de celui-ci, et
- un cannabinoïde, ou un mélange de cannabinoïdes, et
- de l'eau,
dans laquelle la taille des submicroparticules est comprise dans la plage de 600 à 750 nm, le rapport massique du polymère chargé positivement sur le polymère chargé négativement est de 100:1 à 1:100, et dans laquelle les cannabinoïdes sont disposés en plaques hydrophobes formées entre le polymère chargé positivement et les chaînes de polymère chargé négativement.

2. Composition selon la revendication 1, dans laquelle le pH de la composition est de 4 à 7,8, de préférence de 4,5 à 5,2.

3. Composition selon la revendication 1 ou la revendication 2, dans laquelle le rapport massique polymères/cannabinoïde est de 10:1 à 1:10, de préférence de 5:1 à 1:5.

4. Composition selon l'une quelconque des revendications précédentes, dans laquelle le cannabinoïde est un phytocannabinoïde, ou un mélange de phytocannabinoïdes, de préférence sélectionné parmi le cannabidiol, le cannabigérol ou un mélange de ceux-ci.

5. Composition selon l'une quelconque des revendications précédentes, dans laquelle le polymère chargé négativement est un sel d'acide hyaluronique de masse moléculaire de 0,5 à 0,75 MDa, de préférence du hyaluronate de sodium.

6. Composition selon l'une quelconque des revendications précédentes, dans laquelle le polymère chargé positivement est du chitosane d'une masse moléculaire de 0,7 à 1 MDa.

7. Composition selon l'une quelconque des revendications précédentes, dans laquelle l'eau est de l'eau de mer.

8. Composition selon l'une quelconque des revendications précédentes, dans laquelle les submicroparticules ont un indice de polydispersité (PDI) de 0,25 à 0,9, de préférence de 0,35 à 0,5.

9. Procédé de production d'une composition comprenant des submicroparticules de polymère chargées en cannabinoïde et solubles en milieu aqueux, selon l'une quelconque des revendications 1-8, **caractérisé en ce que** ledit procédé comprend les étapes suivantes :
a) l'obtention d'une solution du polymère chargé positivement dissous dans une solution aqueuse acide, ladite solution étant constituée d'acide acétique et d'acétate de sodium, avec un pH ajusté à 4-4,5,
b) l'obtention d'une solution du polymère chargé négativement dissous dans une solution aqueuse acide, ladite solution étant sélectionnée parmi un tampon, et constituée d'acide acétique et d'acétate de sodium, ou d'eau, avec un pH ajusté à 4-4,5,
c) l'obtention d'une solution d'un cannabinoïde, ou d'un mélange de cannabinoïdes en dissolvant lesdits cannabinoïdes dans un solvant sélectionné parmi l'éthanol, le méthanol et l'isopropanol,
d) l'addition de la solution obtenue dans c) à la solution obtenue dans a),
e) l'addition de la solution obtenue dans b) à la solution obtenue dans d), et
f) l'agitation par secouement et l'agitation mécanique ou magnétique de la solution obtenue dans e) jusqu'à ce que le solvant soit évaporé.

10. Procédé selon la revendication 9, dans lequel, dans l'étape a), la solution comprend du chitosane à une concentration de 2 à 6 % (p/p), de préférence de 2,5 à 5 % (p/p), la solution aqueuse acide est constituée d'acide acétique à 0,45 % (p/v) et d'acétate de sodium à 0,55 % (p/v) et le pH est ajusté avec une solution adaptée de HCl 5M et/ou de NaOH 5M.

11. Procédé selon la revendication 9 ou la revendication 10, dans lequel la solution de l'étape b) comprend un sel d'acide hyaluronique à une concentration de 0,5 à 3 % (p/p), de préférence de 0,5 % à 2,5 % (p/p).

12. Procédé selon l'une quelconque des revendications 9-11, dans lequel la solution aqueuse acide de l'étape b) est sélectionnée parmi :
- un tampon constitué d'acide acétique, à une concentration de 0,2% à 0,9% (p/v), et d'acétate de sodium à une concentration de 0,2 % à 0,9 % (p/v), ou
- une solution aqueuse constituée d'eau de mer, dans laquelle la concentration de sels dans l'eau est de 0,1 à 10 % (p/v), de préférence de 3 à 4 % (p/v), avec le pH ajusté par l'addition de HCl.

13. Procédé selon l'une quelconque des revendications 9-12, dans lequel, dans l'étape c), la concentration du cannabinoïde dans le solvant (p/v) est de 0,1 à 50 %, de préférence de 10 à 40 % et plus préférablement de 15 à 30 %.

14. Procédé selon l'une quelconque des revendications 9-13, dans lequel, dans l'étape d), la solution de cannabinoïdes est ajoutée à la solution de l'étape a) dans un rapport (v/v) de 0,04:1 à 0,2:1, de préférence de 0,04:1 à 0,1:1.

15. Procédé selon l'une quelconque des revendications 9-14, dans lequel, dans l'étape e), la solution obtenue dans l'étape b) est ajoutée à la solution obtenue dans l'étape d) dans un rapport (v/v) de 0,5:1 à 1:0,5, de préférence de 0,7:1 à 1:0,7, plus préférablement de 0,8:1 à 1:0,8.

16. Procédé selon l'une quelconque des revendications 9-15, dans lequel, dans l'étape f), la solution est secouée pendant une durée de 30 s à 5 min, de préférence de 30 s à 90 s, puis soumise à une agitation mécanique ou magnétique pendant une durée de 12 à 48 h, de préférence de 12 à 36, et plus préférablement de 18 à 30.

17. Composition nutraceutique, pharmaceutique ou cosmétique comprenant la composition selon l'une quelconque des revendications 1-8.

18. Composition pharmaceutique selon la revendication 17 pour une utilisation dans le traitement des affections cutanées sélectionnées parmi l'acné et la dermatite atopique.

19. Composition pharmaceutique selon la revendication 17 pour une utilisation dans le traitement des troubles oculaires sélectionnés à partir de la rétinopathie diabétique.

20. Utilisation non-thérapeutique de la composition cosmétique, selon la revendication 17, pour des effets relaxants, apaisant et hydratants sur la peau.
